(19) Europäisches Patentamt / European Patent Office / Office européen des brevets

(11) **EP 3 014 501 B1**

(12) **EUROPEAN PATENT SPECIFICATION**

(45) Date of publication and mention of the grant of the patent:
**28.08.2019   Bulletin 2019/35**

(21) Application number: **14735651.3**

(22) Date of filing: **25.06.2014**

(51) Int Cl.:
*G16H 50/30* (2018.01)       *G16H 50/50* (2018.01)
*A61B 5/00* (2006.01)        *A61B 5/04* (2006.01)
*A61B 5/0452* (2006.01)      *A61B 5/0456* (2006.01)

(86) International application number:
**PCT/GB2014/051940**

(87) International publication number:
**WO 2014/207461 (31.12.2014 Gazette 2014/53)**

(54) **MEASURING RESPIRATION**

MESSUNG DER ATMUNG

MESURE DE LA RESPIRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.06.2013   GB 201311498**

(43) Date of publication of application:
**04.05.2016   Bulletin 2016/18**

(73) Proprietor: **Oxford University Innovation Limited Botley Oxford OX2 0JB (GB)**

(72) Inventors:
• **CLIFTON, David Andrew**
  **Oxford**
  **Oxfordshire OX3 7DQ (GB)**
• **PIMENTEL, Marco**
  **Oxford**
  **Oxfordshire OX3 7DQ (GB)**
• **TARASSENKO, Lionel**
  **Oxford**
  **Oxfordshire OX3 7DQ (GB)**

(74) Representative: **J A Kemp**
  **14 South Square**
  **Gray's Inn**
  **London WC1R 5JJ (GB)**

(56) References cited:
  **US-A1- 2013 137 945**

• **CLIFTON L ET AL: "Gaussian Processes for Personalized e-Health Monitoring With Wearable Sensors", IEEE TRANSACTIONS ON BIOMEDICAL ENGINEERING, IEEE SERVICE CENTER, PISCATAWAY, NJ, USA, vol. 60, no. 1, January 2013 (2013-01), pages 193-197, XP011490325, ISSN: 0018-9294, DOI: 10.1109/TBME.2012.2208459**
• **Zitao Liu ET AL: "Modeling Clinical Time Series Using Gaussian Process Sequences" In: "Proceedings of the 2013 SIAM International Conference on Data Mining", 2 May 2013 (2013-05-02), Society for Industrial and Applied Mathematics, Philadelphia, PA, XP055154683, ISBN: 978-1-61-197283-2 pages 623-631, DOI: 10.1137/1.9781611972832.69, abstract section 2.2 Gaussian Processes and Dynamical System Gaussian Process (GP)**
• **SG FLEMING ET AL: "A comparison of signal processing techniques for the extraction of breathing rate from the photoplethysmogram", PROCEEDINGS OF WORLD ACADEMY OF SCIENCE, ENGINEERING AND TECHNOLOGY, WORLD ACADEMY OF SCIENCE, ENGINEERING AND TECHNOLOGY, TURKEY, vol. 24, October 2007 (2007-10), pages 276-280, XP009105542, ISSN: 1307-6884**

- MEREDITH DJ, CLIFTON D, CHARLTON P, BROOKS J, PUGH CW, TARASSENKO L.: "Photoplethysmographic derivation of respiratory rate: a review of relevant physiology", JOURNAL OF MEDICAL ENGINEERING & TECHNOLOGY, vol. 36, no. 1, January 2012 (2012-01), pages 1-7, XP009181456, DOI: 10.3109/03091902.2011.638965
- Carl Edward Rasmussen ET AL: "Gaussian Processes for Machine Learning" In: "Gaussian Processes for Machine Learning", 2006, MIT Press, Berlin, Heidelberg, XP055153328, ISBN: 978-0-26-218253-9 vol. 1 cited in the application chapter 2
- DAVID WONG ET AL: "Probabilistic detection of vital sign abnormality with Gaussian process regression", BIOINFORMATICS&BIOENGINEERING (BIBE), 2012 IEEE 12TH INTERNATIONAL CONFERENCE ON, IEEE, 11 November 2012 (2012-11-11), pages 187-192, XP032296545, DOI: 10.1109/BIBE.2012.6399671 ISBN: 978-1-4673-4357-2
- STEGLE O ET AL: "Gaussian Process Robust Regression for Noisy Heart Rate Data", IEEE TRANSACTIONS ON BIOMEDICAL ENGINEERING, IEEE SERVICE CENTER, PISCATAWAY, NJ, USA, vol. 55, no. 9, 1 September 2008 (2008-09-01), pages 2143-2151, XP011342742, ISSN: 0018-9294, DOI: 10.1109/TBME.2008.923118
- LEI CLIFTON ET AL: "Gaussian process regression in vital-sign early warning systems", ENGINEERING IN MEDICINE AND BIOLOGY SOCIETY (EMBC), 2013 35TH ANNUAL INTERNATIONAL CONFERENCE OF THE IEEE, IEEE, 28 August 2012 (2012-08-28), pages 6161-6164, XP032464340, ISSN: 1557-170X, DOI: 10.1109/EMBC.2012.6347400
- S. Roberts ET AL: "Gaussian processes for time-series modelling", ROYAL SOCIETY OF LONDON. PHILOSOPHICAL TRANSACTIONS.MATHEMATICAL, PHYSICAL AND ENGINEERING SCIENCES, vol. 371, no. 1984, 31 December 2012 (2012-12-31), pages 20110550-20110550, XP055495335, GB ISSN: 1364-503X, DOI: 10.1098/rsta.2011.0550

## Description

The present invention provides an improved way of measuring respiratory rate.

Respiratory rate has been shown to be an important indicator of patient deterioration [4], [11], and extreme values of respiratory rate are associated with an increased risk of adverse events in hospital patients [4], [11], [5]. The importance of assessing respiratory rate has led to its inclusion in most numerical patient assessment systems, often termed early warning scores or EWS [6], the use of which is widespread. Such systems typically consist of the application of univariate scoring criteria to observational physiological variables (including the vital signs) and produce a cumulative score that can, if it exceeds a predefined threshold, lead to identification of physiological deterioration in acutely-ill hospital patients. While automated techniques exist for measuring respiratory rate, they usually require the use of equipment which might interfere with natural breathing, such as spirometry, or might be uncomfortable for the patient, such as measurement via a band that encircles the chest. The signals acquired from conventional methods, including impedance plethysmography (IP), are often unusable as a result of a poor signal-to-noise ratio and are sensitive to frequent movement artefact [12].

The ECG signal, recorded for most acutely ill patients, has been considered as a source of potentially reliable respiratory information. Respiratory activity may cause the ECG to be modulated in two fundamental ways: R-peak amplitude (RPA) modulation, which is caused by the movement of the chest due to the filling and emptying of the lungs (which in turn causes a rotation of the electrical axis of the heart and the consequent modulation of the amplitude of the ECG) [2], and respiratory sinus arrhythmia (RSA), which is a frequency modulation, corresponding to a variation in heart rate that occurs throughout the respiratory cycle [2], [8]. Much previous work exists concerning the estimation of respiratory rate from ECG or other signals, such as the photoplethysmogram or PPG, and the arterial blood pressure (ABP) waveform [18]. These approaches are based on either the RPA- or RSA-modulated signals (or a combination of both), and use a variety of algorithms based on spectral analysis [1], the continuous wavelet transform [3], neural networks [10], and autoregressive models [13], [14]. Small errors (around 1 to 2 breaths per minute) between estimates derived from these signals and reference respiratory rate values have been reported [1], [13], [14] for studies of healthy volunteers.

A drawback with these approaches, however, is that they provide a point estimate of the respiratory rate. The uncertainty associated with the estimated value cannot be directly quantified, due to the nature of the algorithms employed. Experience has shown that existing methods tend to work relatively reliably for healthy volunteers but are less successful for patients who are unwell and/or elderly. In the absence of a robust method of identifying an error associated with the point estimate of respiratory rate, a clinician is unable to distinguish between point estimates that correctly indicate the state of the patient and those that are dominated by noise.

CLIFTON L ET AL, "Gaussian Processes for Personalized e-Health Monitoring With Wearable Sensors", IEEE TRANSACTIONS ON BIOMEDICAL ENGINEERING, IEEE SERVICE CENTER, PISCATAWAY, NJ, USA, vol. 60, no. 1, January 2013, pages 193 - 197 discloses a Gaussian process framework implementing a patient-personalized system for analysis and inference of medical monitoring data in the presence of data uncertainty, typically caused by sensor artifact and data incompleteness.

Zitao Liu ET AL, "Modeling Clinical Time Series Using Gaussian Process Sequences", Proceedings of the 2013 SIAM International Conference on Data Mining, Philadelphia, PA, Society for Industrial and Applied Mathematics, 2 May 2013, pages 623 - 631 discloses a probabilistic approach for modeling clinical time series data that is optimized to handle irregularly sampled observations. The model is defined by a sequence of Gaussian processes (GPs), each restricted to a window of a finite size, where dependencies among two consecutive Gaussian processes are represented using a linear dynamical system.

SG FLEMING ET AL, "A comparison of signal processing techniques for the extraction of breathing rate from the photoplethysmogram", PROCEEDINGS OF WORLD ACADEMY OF SCIENCE, ENGINEERING AND TECHNOLOGY, WORLD ACADEMY OF SCIENCE, ENGINEERING AND TECHNOLOGY, TURKEY, vol. 24, October 2007, pages 276 - 280 discloses a technique for extracting the breathing rate from a PPG waveform using autoregressive modelling.

US 2013/137945 A1 discloses systems and methods for determining the pulse rate of a patient from multiple fiducial points using Gaussian kernel smoothing.

MEREDITH DJ, CLIFTON D, CHARLTON P, BROOKS J, PUGH CW, TARASSENKO L., "Photoplethysmographic derivation of respiratory rate: a review of relevant physiology", JOURNAL OF MEDICAL ENGINEERING & TECHNOLOGY, vol. 36, no. 1, January 2012, pages 1-7 describes the use of PPG waveforms for non-invasive respiratory rate monitoring, the effect of respiration on the PPG waveform, and the key physiological mechanisms that underpin the derivation of respiratory rate from the PPG.

Carl Edward Rasmussen ET AL, "Gaussian Processes for Machine Learning", "Adaptive Computation and Machine Learning", 2006, vol. 1, MIT Press, Berlin, Heidelberg discloses use of Gaussian process methods for regression problems.

DAVID WONG ET AL: "Probabilistic detection of vital sign abnormality with Gaussian process regression", BIOINFORMATICS&BIOENGINEERING (BIBE), 2012 IEEE 12TH INTERNATIONAL CONFERENCE ON, IEEE, 11 November 2012, pages 187-192 describes a Gaussian process regression technique for estimating missing data in the context of vital-sign monitoring and how it can be incorporated within an automated data fusion monitoring system.

STEGLE O ET AL: "Gaussian Process Robust Regression for Noisy Heart Rate Data", IEEE TRANSACTIONS ON BIOMEDICAL ENGINEERING, IEEE SERVICE CENTER, ISCATAWAY, NJ, USA, vol. 55, no. 9, 1 September 2008, pages 2143-2151 discloses a post-processing model for inferring a latent heart rate time series consisting of two main components: unsupervised clustering followed by Bayesian regression. The clustering component uses auxiliary data to learn the structure of outliers and noise bursts. The subsequent Gaussian process regression model uses the cluster assignments as prior information and incorporates expert knowledge about the physiology of the heart.

LEI CLIFTON ET AL: "Gaussian process regression in vital-sign early warning systems", ENGINEERING IN MEDICINE AND BIOLOGY SOCIETY (EMBC), 2013 35TH ANNUAL INTERNATIONAL CONFERENCE OF THE IEEE, IEEE, 28 August 2012, pages 6161 -6164 discloses application of Gaussian process regression machine learning techniques to early warning score (EWS) patient monitoring systems.

S. Roberts ET AL: "Gaussian processes for time-series modelling", ROYAL SOCIETY OF LONDON. PHILOSOPHICAL TRANSACTIONS. MATHEMATICAL, PHYSICAL AND ENGINEERING SCIENCES, vol. 371, no. 1984, 31 December 2012, pages 20110550-20110550 describes Bayesian non-parametric modelling for Gaussian processes.

It is an object of the invention to address at least partially one or more of the shortcomings described above in relation to the prior art.

The scope of the invention is defined by the claims. According to an aspect of the invention, there is provided a method of obtaining information about a respiratory rate according to claim 1.

Thus, an intrinsically probabilistic method is employed that is able to output probabilistic information about respiratory rate. The information may comprise not only an estimate of the respiratory rate but also a statistically derived measure of the uncertainty of the estimate (in contrast to the prior art, which is only able to output a point estimate of the rate). A clinician is therefore able to make a well informed judgement about whether a particular reading is statistically significant (which might prompt the clinician to take action if the reading is outside of a normal range for example) or statistically insignificant (i.e. dominated by noise, in which case the clinician may not react to the reading in the same way). The information may further comprise a probabilistic posterior distribution over the rate (for example as part of a joint distribution over the hyperparameters of the covariance function). This is useful if the output from the method is to be used as the input to a subsequent probabilistic inference system, where knowing the full distribution of the input is more informative than a point estimate.

Choosing a Gaussian Process model and selecting a covariance function that is periodic enables the rate and uncertainty to be extracted directly from an analysis of the joint probability distribution over the hyperparameters of the covariance function. One of the hyperparameters will define the periodicity of the covariance function, which after fitting to the input time series of measurements will correspond to the periodicity of the underlying rate to be measured. By analysing the joint probability distribution over the hyperparameters it is possible to obtain the estimate of the rate and of the uncertainty in the rate by estimating the mode and covariance of the distribution over the hyperparameters.

The use of Gaussian process regression brings all of the advantages of a principled, probabilistic approach: the uncertainty in the estimation is directly quantified; incompleteness, noise, and artefact may be handled in a robust manner; and the output may consist of a predictive posterior distribution, rather than a single estimate. Additionally, due to the generative nature of the approach, it is possible to generate data from the model, which can be useful for estimating the behaviour of respiratory rate during periods of missing data.

According to a further aspect of the invention, there is provided a patient monitor according to claim 5.

The invention may be embodied in computer software adapted to execute the method on a programmed computer system. The computer software may be stored in a portable medium, in memory, or transmitted as a data signal. The programmed computer system may comprise standard computer hardware such as CPU, RAM, etc. that is well known to the skilled person.

The invention will be further described by way of example with reference to the accompanying drawings in which:

Figure 1 is a flow chart illustrating the framework of an example method for obtaining information about the rate of a periodic physiological process;

Figure 2 is a flow chart illustrating an example approach for obtaining the mode and covariance of hyperparameters;

Figure 3 depicts a patient monitor and patient monitoring system;

Figure 4 depicts extraction of respiratory rate from single-lead ECG: (a) ECG signal; (b) Reference (IP) respiration signal; (c) RSA waveform from the R-R intervals time series; (d) RPA waveform from the R-peaks time series; (e) Final respiratory rate estimates from the AR- and GP-based methods;

Figure 5 depicts histograms showing the percentage error $E$ between the proposed method and a "gold standard" reference respiratory rate as follows: data are first partitioned into 1-minute windows. The method is then used to estimate the respiratory rate on each window of data separately. The estimate of respiratory rate for each window is then compared with the reference rate, to give the percentage error $E$, which is shown on the x-axis. The histograms show the number of windows with different ranges of percentage error, *E*, using data for "young" and "elderly" subjects.

According to an embodiment, there is provided a method of obtaining information about the rate of a periodic physiological process from a time series of measurements obtained from a patient. The physiological process is breathing and the rate is the respiratory rate. The time series of measurements may consist of the output from a sensor at a plurality of different times. The output may be selected so as to be sensitive to the physiological process being measured (i.e. so that the physiological process can modulate the signal in a measurable way). Specific examples are mentioned below. The different times at which the time series of measurements are provided may be equally or irregularly spaced. The times series of measurements may comprise the output from plural different sensors at plural different times or plural outputs from the same sensor at each of a plurality of different times.

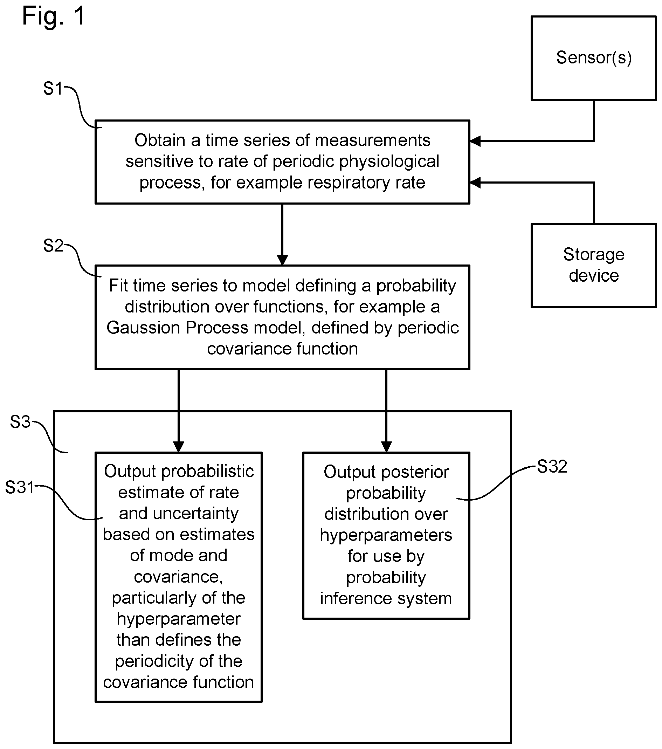

In an embodiment, as illustrated in the flow chart of Figure 1, the method comprises the step (S1) of obtaining the time series of measurements. The time series of measurements may be obtained directly (i.e. substantially in real time) from a sensor on or in close proximity to the patient's body. Alternatively, the times series of measurements may have been obtained by a sensor at an earlier time (e.g. such that all of the time series of measurements was obtained by the sensor and stored before any of the data is used in the method of Figure 1), in which case the time series of measurements may be retrieved from a storage device containing the earlier obtained data (e.g. over a data connection or via direct access to the storage device).

In a subsequent step (S2), a model is fitted to the time series of measurements (this step may also be referred to as a "training" step). The model is of a type that defines a probability distribution over functions. The time series of measurements represents one example of points that occur within such a function. The fitting of the model involves obtaining an estimate of the probability distribution over functions that best fits the time series of measurements. In an embodiment, the fitting involves estimating the mode and covariance of a joint distribution over hyperparameters defining the model.

The fitting process provides information about the respiratory rate, which may be output (step S3) for further processing or display for example. The output may comprise a probability distribution of the rate or an estimate of the rate and an uncertainty. The uncertainty in the rate may be derived from the variance of the distribution over the rate. The model may be characterized by a covariance function that is periodic and the information about the rate may be obtained from an estimate of the distribution of the hyperparameter that defines the periodicity of the covariance function (step S31).

For example, a distribution over all of the hyperparameters defining the model may be obtained from the fitting. The mode of the distribution may be obtained, which will comprise a distinct mode value for each of the hyperparameters considered. An estimate of the rate can then be derived from the mode of the particular hyperparameter which defines the periodicity of the covariance function of the model. The uncertainty in the rate can then be obtained from the variance of that hyperparameter at the mode of the distribution of hyperparameters. The variance of this hyperparameter at the mode can be obtained from the covariance of the distribution over all of the hyperparameters.

Alternatively or additionally, the output step S3 comprises a step (S32) of outputting a posterior probability distribution over the hyperparameters for use, for example, in a probability inference system.

The model is a Gaussian process model and the fitting process is an example of Gaussian process regression.

In an embodiment, the periodic covariance function (i.e. the form of the periodic covariance function) is determined using prior knowledge of the physiological process. The fact that the covariance function is periodic already encodes prior knowledge that the physiological process is periodic. The form of the periodic covariance function may however be configured to encode further prior knowledge that may be available. For example, the periodic covariance function may encode prior knowledge that the rate of the physiological process will drift through time. This may be achieved for example by including a hyperparameter representing a length scale of the periodic covariance function. An example of such a hyperparameter is the hyperparameter $\lambda$ discussed below in the context of the "detailed example". Modifying the periodic covariance function to include prior knowledge may improve (e.g. in terms of efficiency and/or accuracy) the fitting of the model to the time series of measurements.

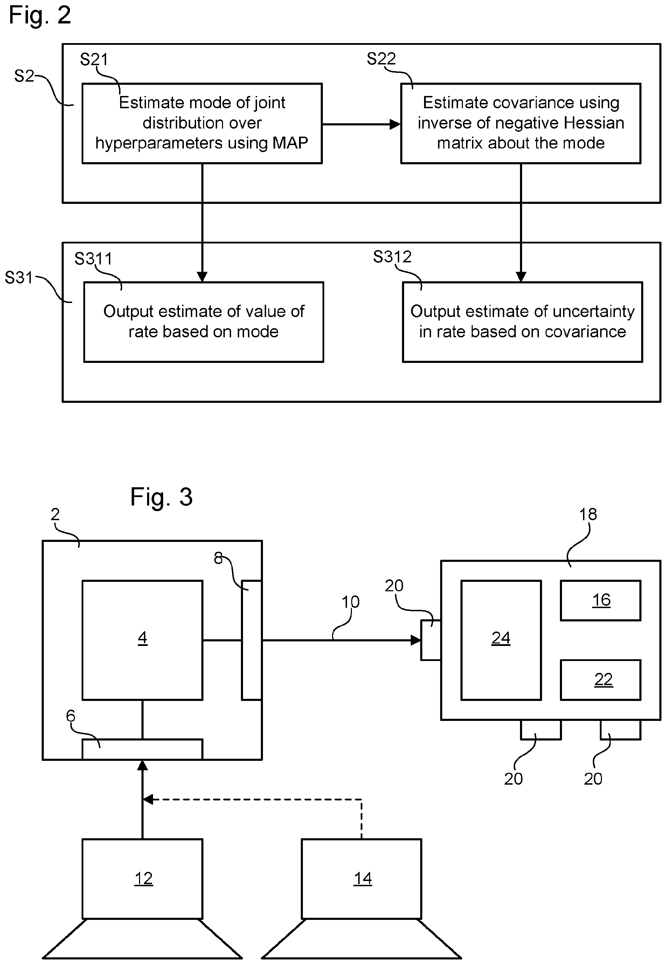

Figure 2 illustrates in further detail how the fitting/training step S2 and output step S31 may be configured in a particular embodiment. According to this embodiment, the fitting/training step S2 comprises a step S21, in which an estimate of the mode of a joint distribution over the hyperparameters is obtained using a maximum a posteriori (MAP) estimate. This mode is then used to output an estimate of the rate (step S311) as part of the output step S31. According to this embodiment, the fitting/training step S2 further comprises a step S22, in which the covariance is obtained using the inverse of the negative Hessian matrix about the mode obtained in step S21. The covariance can then be used to derive a quantitative measure of the uncertainty in the rate value output in step S311, which may be output in step S312.

Figure 3 depicts an example of apparatus that could be configured to implement a method according to an embodiment. The depicted apparatus comprises a patient monitor 2 comprising a data processor 4 (comprising for example, CPU, motherboard, RAM, etc.) configured to carry out the data processing operations necessary to implement the method of obtaining information about the rate of the periodic physiological process according to an embodiment.

The patient monitor 2 shown comprises an I/O interface 6 for receiving data from one or more sensors 12,14, or from a data source, in order to obtain the required time series of measurement data that is used for fitting to the model (e.g. using Gaussian process regression). The sensor(s) 12,14 may be adapted to obtain photoplethysmogram data (from either a pulse oximeter or from video camera recordings of a patient's skin), arterial blood pressure waveform data and/or ECG data for example.

The patient monitor 2 comprises a further I/O interface 8 for outputting information about the rate that has been derived from the input times series of measurement data. In the particular embodiment shown, the output data 10 is transmitted (e.g. via a network connection) to an input interface 20 of a patient monitoring system 18. The patient monitoring system 18 may be configured to store the data output from the patient monitor 2 in memory 16, for example, and/or display the data on display 22. In an embodiment, the patient monitoring system 18 comprises a probabilistic inference system 24 (which may comprise standard computer hardware programmed for carrying out a probabilistic inference method) and the patient monitor 8 may be configured to output a probabilistic posterior distribution over the rate. The probabilistic inference system 24 may be configured for example to detect an abnormal state of the patient by combining the output 10 from the patient monitor 2 with one or more further probabilistic information inputs (input via an I/O interface 20 for example) derived from other measurements performed on the patient.

## DETAILED EXAMPLE

The presence of respiratory information within the electrocardiogram (ECG) signal is a well-documented phenomenon. In the detailed example described below, a Gaussian process framework is used for the estimation of respiratory rate from different sources of modulation in a single-lead ECG. A periodic covariance function is used to model the frequency- and amplitude-modulated time series measurements derived from the ECG, where the hyperparameters of the process are used to derive the respiratory rate. In the particular example described, the approach is evaluated using data taken from 40 healthy subjects each with 2 hours of monitoring, containing ECG and respiration (impedance plethysmography, or IP) waveforms. Results indicate that the accuracy of the example method is comparable with that of existing methods, which are non-probabilistic, but with the advantages of the example method being a principled probabilistic approach, including the direct quantification of the uncertainty in the estimation.

To illustrate the efficacy of an embodiment of the invention, the method was applied to data from the Physiobank/Physionet Fantasia database [7], [9]. The latter consists of data acquired from two subgroups of volunteers: 20 "young" (21 - 34 years old) and 20 "elderly" (60 - 85 years old) healthy subjects who underwent 120 minutes of continuous supine rest (while watching the film "Fantasia"). Continuous single-lead ECG and respiration (IP) signals were collected. Each subgroup of subjects includes equal numbers of men and women.

Respiratory rate was computed using methods to be described later, with windows of data of 1 minute duration, with successive windows having 50s overlap (i.e., a new estimate is produced every 10s).

### A. Extracting the respiratory waveforms

ECG beat detection was performed using the Hamilton and Tompkins algorithm [15]. The amplitude of the resulting series of R-peaks was determined in order to derive the RPA waveform. The intervals between successive R-peaks were also calculated to derive the R-R time series, which corresponds to the RSA waveform.

### B. Calculation of respiratory rate from the RPA and RSA waveforms

Respiratory rate is extracted from the RPA and RSA waveforms using Gaussian process (GP) regression. This offers a framework for performing inference using time-series data, in which a probability distribution over a functional space is constructed. We consider the RPA and RSA waveforms to be two separate functions, from which we can perform inference using the GP framework. In comparison with many prior art approaches based on algorithms such as spectral analysis, continuous wavelet transforms or autoregressive models, the GP framework can be applied efficiently to the analysis of data that may be sampled at irregular intervals.

The GP is a stochastic process [16] that expresses a dependent variable $y$ in terms of an independent variable $x$, via a latent function $f(x)$. This function can be interpreted as being a probability distribution over functions,

$$\mathbf{y} = f(\mathbf{x}) \sim GP(m(\mathbf{x}), k(\mathbf{x}, \mathbf{x}'))$$

where $m(\mathbf{x})$ is the mean function of the distribution and $k$ is a covariance function which describes the coupling between two values of the independent variable as a function of the (kernel) distance between them. The covariance function encodes our assumptions concerning the structure of the time series that we wish to model [16]. Valid covariance functions can take a variety of forms, with the constraint that they are positive semi-definite.

In the present example, **x**, **y** will be the times of the observations and the values of the observations, respectively, comprising univariate pairs $(x, y)$. A periodic covariance function is defined as follows, denoted $r = \|x_p - x_q\|$ as the (Euclidean) distance between two independent variables, $x_p$ and $x_q$:

$$k(r) = \sigma_0^2 \exp\left[ -\frac{\sin^2((2\pi / P_L)r)}{2\lambda^2} \right],$$

in which the hyperparameters $\sigma_0$ and $\lambda$ give the amplitude and length-scale of the latent function, respectively. $P_L$ is the length of the period of the covariance function and provides a direct estimate of the respiratory rate. The selection of a covariance function k that is periodic represents the prior knowledge of typical waveforms from which respiration may be derived, which are expected to be periodic. It is assumed that the observations are corrupted by additive Gaussian noise with variance component $\varepsilon^2$. Thus, the full covariance function is given by

$$V(x_p, x_q) = k(x_p, x_q) + \varepsilon^2 \delta\left(\left\| x_p - x_q \right\|\right)$$

where $\delta$ is the Kronecker delta, for which $\delta = 1$ if $p = q$, and $\delta = 0$ otherwise. Here, $\varepsilon$ is the noise variance. The values of the hyperparameters are learned from univariate respiration waveforms which consist of $n$ observations, $D = \{(x_i, y_i)\}|i=1,...,n\}$. The $x_i$ and $y_i$ points represent the independent and dependent variable values respectively.

The nature of the GP is such that, conditional on observed data, predictions can be made about the function values $f(x_*)$ at any ("test") location of the index set $x_*$. The distribution of the values of $f$ at point $x_*$ is Gaussian, with mean and covariance given by

$$f_* \Big| x_*, \mathbf{x}, \mathbf{y} \sim \mathbf{N}(\overline{f_*}, Var[f_*])$$

in which **x**, **y** are the "training data", $D$, and where **N** denotes the Normal or Gaussian distribution with mean and variance parameters. The above makes it possible to determine the following predictive equations for GP regression, for which it is assumed that the mean function m is zero,

$$\overline{f}_* = m(x_*) + \mathbf{k}(x_*, x_*)^\top \mathbf{V}(\mathbf{x}, \mathbf{x})^{-1}(\mathbf{y} - m(\mathbf{x}))$$

$$Var[f_*] = \mathbf{k}(x_*, x_*) - \mathbf{k}(\mathbf{x}, x_*)^\top \mathbf{V}(\mathbf{x}, \mathbf{x})^{-1}\mathbf{k}(\mathbf{x}, x_*)$$

In the above, boldface terms **k**, **V** refer to the matrix equivalents of $k$, $V$ defined earlier. In the present example, for the particular dataset considered, empirical selection of suitable prior values of the hyperparameters were $\sigma_0 = 1$, $\lambda = 1$ and $\varepsilon = 0.01$. For the period $P_L$, a uniform prior distribution was selected over the range $P_L = (1.5...10)$, which corresponds to plausible respiratory rate values of 6 to 40 breaths per minute.

A full Bayesian treatment of GP regression requires integration over the posterior distribution of the hyperparameters. Even though most calculations in the GP regression framework are analytically tractable, the integral over the posterior of the hyperparameters often is not. The integration over the posterior of the hyperparameters $p(\theta|D)$, with hyperparameters $\theta = \{\sigma_0, \lambda, P_L, \varepsilon\}$, can be approximated by a point via the maximum a posteriori (MAP) estimate

$$\hat{\theta} = \arg\max_{\theta} p(\theta|D)$$

$$= \arg\min_{\theta} \left[ -\log p(D|\theta) - \log p(\theta) \right]$$

In this approximation, the distribution over the hyperparameters is assigned a point mass at the mode of the posterior, allowing the marginal distribution of the latent function to be approximated by $p(f|D) \approx p(f|D, \hat{\theta})$. This approach is com-

putationally attractive. The grid search approximation to the full integral over the posterior distributions of the hyperparameters follows the work of Rue et al. [17], in which the posterior mode $\hat{\theta}$ is first located by maximising the log-posterior distribution log $p(\theta|y)$, and the shape of the log-posterior is approximated with a Gaussian, the covariance of which is the inverse of the negative Hessian at the mode (more details may be found in [16], [17]).

In the present example, for each 60s window, the RPA and RSA time series are first normalized using a zero mean, unit-variance transformation. A Gaussian process is then fitted to each of the waveforms, using the procedure described above to obtain an estimate of both the value and uncertainty of the respiratory rate value (directly from the distribution over the period, $P_L$). The estimate with lower uncertainty (i.e., where the posterior distribution had lowest variance) was chosen as the final estimate of the respiratory rate for that window.

The performance of the present example was compared with that of the autoregressive model, a parametric, non-probabilistic spectral analysis technique that has been successively applied to this problem [13], [14]. This method requires equispaced samples, and so the time series of R-R intervals and R-peaks were resampled at 4 Hz, and the RPA and RSA waveforms were obtained using linear interpolation. Each of the waveforms was then filtered using an FIR band-pass filter with cut-off frequencies of 0.1 and 0.6 Hz (equivalent to respiratory rates of 6-36 breaths per min). Following previous methods, the steps involved in estimating respiratory rates are as follows for each of the RPA and RSA waveforms: (i) fit an AR model to each 60 s window of resampled data; (ii) reject all poles with frequencies corresponding to respiratory rates outside the range of physiologically-plausible values (6-36 breaths per min); (iii) keep all poles with magnitude of at least 95% of the remaining highest-magnitude pole; and (iv) select the pole remaining that has the smallest angle. The frequency associated with this pole is the estimate of respiratory rate for this waveform. Finally, the respiratory rate corresponding to the pole with the highest magnitude (extracted from the two waveforms) was selected as the final respiratory rate for that window.

*C. Reference respiratory rate*

A reference respiratory rate was calculated from the IP signal in the database using both an extrema detection algorithm and an AR-based method. With the latter, the respiration signal was down-sampled to 4 Hz, after applying an anti-aliasing filter, and a 0.1-0.6 Hz FIR band-pass filter was then applied. The resulting waveform was then fitted to an AR model and the respiratory pole identified using the same method as described in the previous section. Only those reference waveforms for which the agreement between both extrema- and AR-based estimates was within 3 breaths per min were retained ("valid windows"). As a result, 72% of the available windows were deemed to be "valid". This approach ensures only the highest quality reference values are considered by potentially eliminating regions of low IP quality.

*Results*

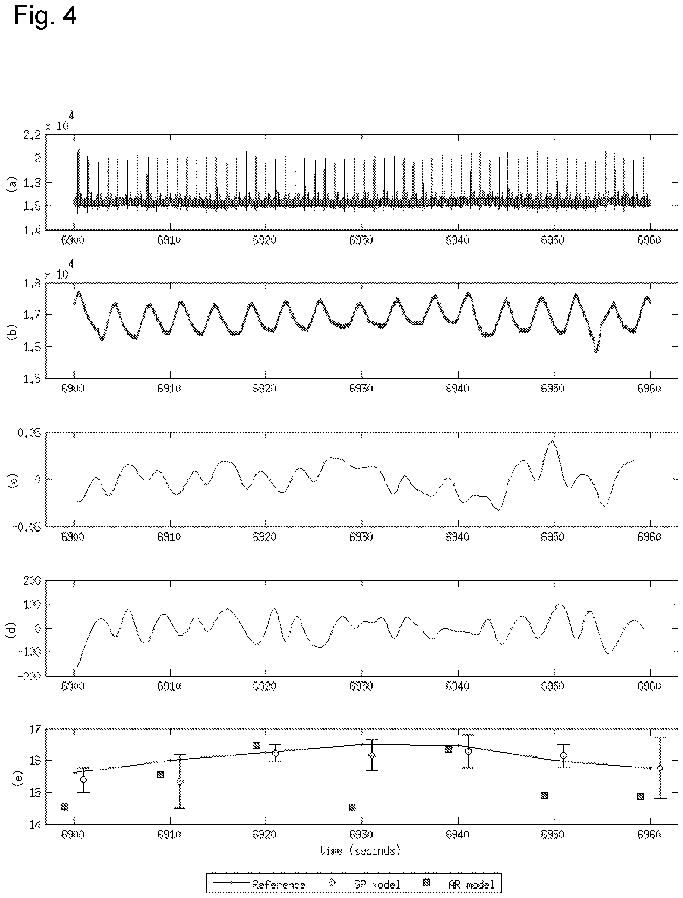

Over the entire database, the mean reference respiratory rate was 18.3 $\pm$ 2.9 breaths per min (17.9 $\pm$ 2.8 for the young subjects and 18.8 $\pm$ 3.0 for the elderly subjects). Figure 4 shows an example from a 1-minute window of data. In general, as illustrated in the figure, it can be seen that the values extracted using the AR method and the proposed GP method are close to the corresponding reference respiratory rate. However, using the GP-based approach, it is possible explicitly to quantify the uncertainty in the estimated value by computing the variance of the posterior distribution drawn from the related period length parameter ($P_L$) of the GP. The uncertainty of an estimate may be due to the presence of noise in the derived respiration waveform (which in turn may be caused by a bad performance of the beat detector), which precludes an accurate estimation of the respiratory rate. In such cases, the precision (inverse of the variance) of the estimate is very low.

The performances of the AR and proposed methods were assessed by calculating the mean absolute error

$$MAE = \frac{1}{n}\sum\nolimits_{i=1}^{n}\left|\hat{y}_i - y_{ref,i}\right|,$$

(*MAE*) in breaths per min, where $n$ is the number of valid windows over the entire database of both groups (young and elderly subjects), $\hat{y}_i$ is the estimated respiratory rate (mean posterior value in the case of the GP-based method) $y_{ref,i}$ is the reference respiratory rate for window $i$. Table I shows *MAE* for different ranges of respiratory rates.

TABLE I

MEAN ABSOLUTE ERROR IN BREATHS PER MIN (BPM)

| Range of reference RR | Young subjects | | Elderly subjects | |
|---|---|---|---|---|
| | AR | GP | AR | GP |
| All data | 1.26 | 1.31 | 1.62 | 1.59 |
| < 12 bpm | 1.66 | 1.68 | 1.52 | 1.57 |
| 12 - 16 bpm | 1.09 | 1.21 | 1.35 | 1.29 |
| 16 - 20 bpm | 1.21 | 1.19 | 1.12 | 1.22 |
| > 20 bpm | 1.90 | 1.71 | 2.09 | 1.82 |

While both methods show similar performance, both performed better for the young group of patients. As the respiratory rate increases (or decreases), the estimation errors also increase. Crucially, it can be seen that the method disclosed herein is more accurate for higher respiration rates in the elderly, which is the typical target population. To assess further the performance of the method, the percentage of valid windows for different ranges of the percentage

$$E = \frac{MAE}{|\mu_{ref}|} \times 100 \,,$$

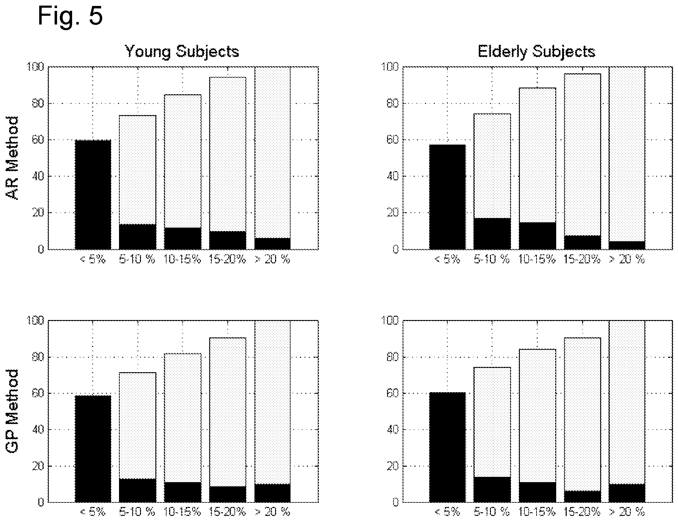

error were calculated (see Figure 5), which is given by                     where $\mu_{ref}$ is the mean of the reference respiratory rates over each of the two patient groups. This is a useful metric since the significance of $MAE$ is different depending on the actual respiratory rate. It can be seen that both methods perform similarly.

Thus, a novel probabilistic approach for extracting respiratory rate from time-series sensor data using Gaussian processes is provided. The method is able to give not only a point estimate of the breathing rate, but, for the first time, a measure of uncertainty of the estimate. By applying this technique to a database of 40 healthy subjects, it has been demonstrated that it is possible to match the performance of the existing state-of-the-art, while bringing the benefits of a probabilistic framework.

REFERENCES

[1] K.H. Chon, S. Dash, and K. Ju. Estimation of respiratory rate from photoplethysmogram data using time-frequency spectral estimation. IEEE Transactions on Biomedical Engineering, 56(8):2054-2063, 2009.

[2] G.D. Clifford, F. Azuaje, P. McSharry, et al. Advanced methods and tools for ECG data analysis. Artech House, 2006.

[3] D. Clifton, J.G. Douglas, P.S. Addison, and J.N. Watson. Measurement of respiratory rate from the photoplethysmogram in chest clinic patients. Journal of clinical monitoring and computing, 21(1):55-61, 2007.

[4] M. Cretikos, J. Chen, K. Hillman, R. Bellomo, S. Finfer, A. Flabouris, et al. The objective medical emergency team activation criteria: A case-control study. Resuscitation, 73(1):62-72, 2007.

[5] J.F. Fieselmann, M.S. Hendryx, C.M. Helms, and D.S. Wakefield. Respiratory rate predicts cardiopulmonary arrest for internal medicine inpatients. Journal of general internal medicine, 8(7):354-360, 1993.

[6] H. Gao, A. McDonnell, D.A. Harrison, T. Moore, S. Adam, K. Daly, L. Esmonde, D.R. Goldhill, G.J. Parry, A. Rashidian, et al. Systematic review and evaluation of physiological track and trigger warning systems for identifying at-risk patients on the ward. Intensive care medicine, 33(4):667-679, 2007.

[7] Goldberger, A. L. et al. Physiobank, physiotoolkit, and physionet. Circulation, 101(23):e215-e220, 2000 (June 13).

[8] JA Hirsch and B. Bishop. Respiratory sinus arrhythmia in humans: how breathing pattern modulates heart rate. American Journal of Physiology-Heart and Circulatory Physiology, 241(4):H620-H629, 1981.

[9] N. Iyengar, CK Peng, R. Morin, AL Goldberger, and L.A. Lipsitz. Age-related alterations in the fractal scaling of cardiac interbeat interval dynamics. American Journal of Physiology-Regulatory, Integrative and Comparative Physiology, 271(4):R1078-R1084, 1996.

[10] A. Johansson. Neural network for photoplethysmographic respiratory rate monitoring. Medical and Biological Engineering and Computing, 41(3):242-248, 2003.

[11] J. Kause, G. Smith, D. Prytherch, M. Parr, A. Flabouris, K. Hillman, et al. A Comparison of Antecedents to Cardiac Arrests, Deaths and Emergency Intensive Care Admissions in Australia and New Zealand, and the United Kingdom the ACADEMIA study. Resuscitation, 62(3):275-282, 2004.

[12] V.H. Larsen, P.H. Christensen, H. Oxhøj, and T. Brask. Impedance pneumography for long-term monitoring of respiration during sleep in adult males. Clinical Physiology, 4(4):333-342, 1984.

[13] J. Lee and KH Chon. Respiratory rate extraction via an autoregressive model using the optimal parameter search criterion. Annals of biomedical engineering, 38(10):3218-3225, 2010.

[14] C. Orphanidou, S. Fleming, SA Shah, and L. Tarassenko. Data fusion for estimating respiratory rate from a single-lead ecg. Biomedical Signal Processing and Control, 2012.

[15] J. Pan and W.J. Tompkins. A real-time QRS detection algorithm. Biomedical Engineering, IEEE Transactions on, (3):230-236, 1985.

[16] C.E. Rasmussen and C.K.I. Williams. Gaussian processes for machine learning, volume 1. MIT press Cambridge, MA, 2006.

[17] H. Rue, S. Martino, and N. Chopin. Approximate Bayesian inference for latent Gaussian models by using integrated nested Laplace approximations. Journal of the royal statistical society: Series b (statistical methodology), 71(2):319-392, 2009.

[18] N. Shamim, M. Atul, C. Gari D, et al. Data fusion for improved respiration rate estimation. EURASIP Journal on advances in signal processing, 2010, 2010.

**Claims**

1. A computer-implemented method of obtaining information about a respiratory rate from a time series of measurements obtained from a patient, comprising:

   obtaining the time series of measurements, the time series of measurements comprising RPA and RSA waveforms obtained from an ECG signal; and

   using Gaussian regression to fit a Gaussian process to each of the RPA and RSA waveforms, the Gaussian process being a stochastic process that expresses a dependent variable in terms of an independent variable via a latent function which can be interpreted as being a probability distribution over functions, the Gaussian process being defined by a mean function and a periodic covariance function, wherein:

   for each of the RPA and RSA waveforms the fitting of the Gaussian process is used to obtain an estimate of the respiratory rate and an estimate of the uncertainty in the respiratory rate directly from the distribution over the hyperparameter defining the period of the covariance function, the hyperparameter being equal or directly proportional to the period of breathing corresponding to the respiratory rate,

   each estimate of the respiratory rate is obtained from the estimated mode of the distribution over the hyperparameter defining the period of the covariance function,

   each estimate of the uncertainty in the estimated respiratory rate is obtained from the variance of the distribution over the hyperparameter defining the period of the covariance function at the mode, and

   the method further comprises selecting and outputting the estimate of the respiratory rate for which the variance of the distribution is lowest as the estimate of the respiratory rate having the lower uncertainty.

2. A method according to claim 1, wherein the form of the periodic covariance function is determined using prior knowledge of the physiological process.

3. A method according to any of the preceding claims, further comprising outputting a probabilistic posterior distribution over the respiratory rate.

4. A method according to any of the preceding claims, wherein the covariance function is defined as follows:

$$V(x_p, x_q) = k(x_p, x_q) + \varepsilon^2 \delta(\|x_p - x_q\|)$$

with k being given as follows:

$$k(r) = \sigma_0^2 \exp\left[-\frac{\sin^2((2\pi/P_L)r)}{2\lambda^2}\right]$$

and in which the hyperparameters are $\sigma_0$, $\lambda$, $P_L$, and $\varepsilon$, where $\sigma_0$ and $\lambda$ give the amplitude and length-scale of the latent function, respectively, $P_L$ is the hyperparameter defining the period of the covariance function, $\varepsilon^2$ represents the variance of an additive noise component, wherein $\delta$ is the Kronecker delta, for which $\delta = 1$ if $p = q$, and $\delta = 0$ otherwise, and $x_p$ and $x_q$ represent two independent variables, and wherein $r = \|x_p - x_q\|$ is the Euclidean distance between the two independent variables.

5. A patient monitor comprising:

   a sensor for obtaining an ECG signal; and
   a data processor adapted to execute the method of any one of the preceding claims to obtain information about the respiratory rate from the ECG signal.

6. A computer program comprising program code means for executing on a programmed computer system the method of any one of claims 1 to 4.

**Patentansprüche**

1. Computerimplementiertes Verfahren des Erhaltens von Informationen über eine Atemfrequenz von einer Zeitreihe von Messungen, die von einem Patienten erhalten werden, umfassend:

   Erhalten der Zeitreihe von Messungen, wobei die Zeitreihe von Messungen RPA- und RSA-Wellenformen umfasst, die von einem EKG-Signal erhalten werden; und
   Verwenden von Gauß-Prozess-Regression, um einen Gauß-Prozess jeder der RPA- und RSA-Wellenformen anzupassen, wobei der Gauß-Prozess ein stochastischer Prozess ist, der eine abhängige Variable in Bezug auf eine unabhängige Variable über eine latente Funktion ausdrückt, die als eine Wahrscheinlichkeitsverteilung über Funktionen interpretiert werden kann, wobei der Gauß-Prozess durch eine Mittelfunktion und eine periodische Kovarianzfunktion definiert ist, wobei:

      für jede der RPA- und RSA-Wellenformen das Anpassen des Gauß-Prozesses verwendet wird, um eine Schätzung der Atemfrequenz und eine Schätzung der Ungewissheit bei der Atemfrequenz unmittelbar von der Verteilung über den Hyperparameter zu erhalten, der den Zeitraum der Kovarianzfunktion definiert, wobei der Hyperparameter gleich wie oder unmittelbar proportional zu dem Zeitraum des Atmens entsprechend der Atemfrequenz ist,
      jede Schätzung der Atemfrequenz von dem geschätzten Modus der Verteilung über den Hyperparameter erhalten wird, der den Zeitraum der Kovarianzfunktion definiert,
      jede Schätzung der Ungewissheit bei der geschätzten Atemfrequenz von der Varianz der Verteilung über den Hyperparameter erhalten wird, der den Zeitraum der Kovarianzfunktion an dem Modus definiert, und
      das Verfahren ferner das Auswählen und Ausgeben der Schätzung der Atemfrequenz umfasst, für die die Varianz der Verteilung am niedrigsten ist, wenn die Schätzung der Atemfrequenz die niedrigere Ungewissheit hat.

2. Verfahren nach Anspruch 1, wobei die Form der periodischen Kovarianzfunktion unter Verwendung von Vorwissen über den physiologischen Vorgang bestimmt wird.

3. Verfahren nach einem der vorhergehenden Ansprüche, ferner umfassend das Ausgeben einer probabilistische A-posteriori-Verteilung über der Atemfrequenz.

4. Verfahren nach einem der vorhergehenden Ansprüche, wobei die Kovarianzfunktion wie folgt definiert ist:

$$V(x_p, x_q) = k(x_p, x_q) + \varepsilon^2 \delta\left(\left\|x_p - x_q\right\|\right)$$

wobei k wie folgt angegeben ist:

$$k(r) = \sigma_0^2 \exp\left[ -\frac{\sin^2((2\pi / P_L)r)}{2\lambda^2} \right]$$

und wobei die Hyperparameter $\sigma_0$, $\lambda$, $P_L$ und $\varepsilon$ sind, wobei $\sigma_0$ und $\lambda$ die Amplitude bzw. die Längenskala der latenten Funktion angeben, $P_L$ der Hyperparameter ist, der den Zeitraum der Kovarianzfunktion definiert, $\varepsilon^2$ die Varianz eines zusätzlichen Rauschanteils darstellt, wobei $\delta$ das Kronecker-Delta ist, für das $\delta = 1$ gilt, wenn $p = q$ und ansonsten $\delta = 0$ gilt, und $x_p$ und $x_q$ zwei unabhängige Variablen darstellen und wobei $r = \|x_p - x_q\|$ der Euklidische Abstand zwischen den zwei unabhängigen Variablen ist.

5. Patientenüberwachungsgerät, umfassend:

   einen Sensor zum Erhalten eines EKG-Signals; und
   einen Datenprozessor, der angepasst ist, um das Verfahren nach einem der vorhergehenden Ansprüche auszuführen, um Informationen über die Atemfrequenz von dem EKG-Signal zu erhalten.

6. Computerprogramm, umfassend Programmcodemittel zum Ausführen des Verfahrens nach einem der Ansprüche 1 bis 4 auf einem programmierten Computersystem.

## Revendications

1. Procédé informatisé d'obtention d'informations sur une fréquence respiratoire à partir d'une série temporelle de mesures obtenues auprès d'un patient, comprenant :

   l'obtention de la série temporelle de mesures, la série temporelle de mesures comprenant des formes d'onde de RPA (amplitude respiratoire de crête) et RSA (arythmie sinusale respiratoire) obtenues à partir d'un signal d'électrocardiogramme (ECG) ; et
   l'utilisation d'une régression gaussienne pour adapter un processus gaussien à chacune des formes d'onde de RPA et RSA, le processus gaussien étant un processus stochastique qui exprime une variable dépendante en termes de variable indépendante par le biais d'une fonction latente qui peut être interprétée comme constituant une répartition de probabilités sur des fonctions, le processus gaussien étant défini par une fonction moyenne et une fonction de covariance périodique,
   pour chacune des formes d'onde de RPA et RSA, l'adaptation du processus gaussien étant utilisée pour obtenir une estimation de la fréquence respiratoire et une estimation de l'incertitude de la fréquence respiratoire directement à partir de la répartition sur l'hyperparamètre définissant la période de la fonction de covariance, l'hyperparamètre étant égal ou directement proportionnel à la période de respiration correspondant à la fréquence respiratoire,
   chaque estimation de la fréquence respiratoire étant obtenue à partir du mode estimé de la répartition sur l'hyperparamètre définissant la période de la fonction de covariance,
   chaque estimation de l'incertitude de la fréquence respiratoire estimée étant obtenue à partir de la variance de la répartition sur l'hyperparamètre définissant la période de la fonction de covariance au niveau du mode, et
   le procédé comprenant en outre la sélection et l'émission de l'estimation de la fréquence respiratoire pour laquelle la variance de la répartition est la moins élevée en tant qu'estimation de la fréquence respiratoire présentant l'incertitude inférieure.

2. Procédé selon la revendication 1, dans lequel la forme de la fonction de covariance périodique est déterminée à l'aide d'une connaissance préalable du processus physiologique.

3. Procédé selon l'une quelconque des revendications précédentes, comprenant en outre l'émission d'une répartition postérieure probabiliste sur la fréquence respiratoire.

4. Procédé selon l'une quelconque des revendications précédentes, dans lequel la fonction de covariance est définie comme suit :

$$V(x_p, x_q) = k(x_p, x_q) + \varepsilon^2 \delta(\|x_p - x_q\|)$$

avec k étant donné comme suit :

$$k(r) = \sigma_0^2 \exp\left[-\frac{\sin^2((2\pi/P_L)r)}{2\lambda^2}\right]$$

et où les hyperparamètres sont $\sigma_0$, $\lambda$, $P_L$ et $\varepsilon$, où $\sigma_0$ et $\lambda$ donnent l'amplitude et une échelle de longueur de la fonction latente, respectivement, $P_L$ est l'hyperparamètre définissant la période de la fonction de covariance, $\varepsilon^2$ représente la variance d'une composante de bruit supplémentaire, $\delta$ étant le delta de Kronecker, pour lequel $\delta = 1$ si $p = q$, et $\delta = 0$ sinon, et $x_p$ et $x_q$ représentent deux variables indépendantes, et r = $\|x_p - x_q\|$ étant la distance euclidienne entre les deux variables indépendantes.

5. Moniteur de patient comprenant :

un capteur permettant d'obtenir un signal d'ECG ; et
un processeur de données conçu pour effectuer le procédé selon l'une quelconque des revendications précédentes pour obtenir des informations sur la fréquence respiratoire à partir du signal d'ECG.

6. Programme informatique comprenant un moyen de code de programme destiné à effectuer sur un système informatique programmé le procédé selon l'une quelconque des revendications 1 à 4.

## Fig. 1

Sensor(s)

S1 — **Obtain a time series of measurements sensitive to rate of periodic physiological process, for example respiratory rate**

Storage device

S2 — **Fit time series to model defining a probability distribution over functions, for example a Gaussion Process model, defined by periodic covariance function**

S3

S31 — **Output probabilistic estimate of rate and uncertainty based on estimates of mode and covariance, particularly of the hyperparameter than defines the periodicity of the covariance function**

**Output posterior probability distribution over hyperparameters for use by probability inference system** — S32

## Fig. 2

S2

**S21** — Estimate mode of joint distribution over hyperparameters using MAP

**S22** — Estimate covariance using inverse of negative Hessian matrix about the mode

S31

**S311** — Output estimate of value of rate based on mode

**S312** — Output estimate of uncertainty in rate based on covariance

## Fig. 3

# Fig. 4

## Fig. 5

# EP 3 014 501 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2013137945 A1

### Non-patent literature cited in the description

- Gaussian Processes for Personalized e-Health Monitoring With Wearable Sensors. **CLIFTON L et al.** IEEE TRANSACTIONS ON BIOMEDICAL ENGINEERING. IEEE SERVICE CENTER, January 2013, vol. 60, 193-197
- Modeling Clinical Time Series Using Gaussian Process Sequences. **ZITAO LIU et al.** Proceedings of the 2013 SIAM International Conference on Data Mining. Society for Industrial and Applied Mathematics, 02 May 2013, 623-631
- A comparison of signal processing techniques for the extraction of breathing rate from the photoplethysmogram. **SG FLEMING et al.** PROCEEDINGS OF WORLD ACADEMY OF SCIENCE, ENGINEERING AND TECHNOLOGY, WORLD ACADEMY OF SCIENCE, ENGINEERING AND TECHNOLOGY, TURKEY. October 2007, vol. 24, 276-280
- **MEREDITH DJ ; CLIFTON D ; CHARLTON P ; BROOKS J ; PUGH CW ; TARASSENKO L.** Photoplethysmographic derivation of respiratory rate: a review of relevant physiology. *JOURNAL OF MEDICAL ENGINEERING & TECHNOLOGY,* January 2012, vol. 36 (1), 1-7
- Gaussian Processes for Machine Learning. **CARL EDWARD RASMUSSEN et al.** Adaptive Computation and Machine Learning. MIT Press, 2006, vol. 1
- Probabilistic detection of vital sign abnormality with Gaussian process regression. **DAVID WONG et al.** BIOINFORMATICS&BIOENGINEERING (BIBE), 2012 IEEE 12TH INTERNATIONAL CONFERENCE ON. IEEE, 11 November 2012, 187-192
- Gaussian Process Robust Regression for Noisy Heart Rate Data. **STEGLE O et al.** IEEE TRANSACTIONS ON BIOMEDICAL ENGINEERING. IEEE SERVICE CENTER, 01 September 2008, vol. 55, 2143-2151
- Gaussian process regression in vital-sign early warning systems. **LEI CLIFTON et al.** ENGINEERING IN MEDICINE AND BIOLOGY SOCIETY (EMBC), 2013 35TH ANNUAL INTERNATIONAL CONFERENCE OF THE IEEE. IEEE, 28 August 2012, 6161-6164
- Gaussian processes for time-series modelling. **S. ROBERTS et al.** ROYAL SOCIETY OF LONDON. PHILOSOPHICAL TRANSACTIONS. MATHEMATICAL, PHYSICAL AND ENGINEERING SCIENCES. 31 December 2012, vol. 371, 20110550-20110550
- **K.H. CHON ; S. DASH ; K. JU.** Estimation of respiratory rate from photoplethysmogram data using time-frequency spectral estimation. *IEEE Transactions on Biomedical Engineering,* 2009, vol. 56 (8), 2054-2063
- **G.D. CLIFFORD ; F. AZUAJE ; P. MCSHARRY et al.** Advanced methods and tools for ECG data analysis. *Artech House,* 2006
- **D. CLIFTON ; J.G. DOUGLAS ; P.S. ADDISON ; J.N. WATSON.** Measurement of respiratory rate from the photoplethysmogram in chest clinic patients. *Journal of clinical monitoring and computing,* 2007, vol. 21 (1), 55-61
- **M. CRETIKOS ; J. CHEN ; K. HILLMAN ; R. BELLOMO ; S. FINFER ; A. FLABOURIS et al.** The objective medical emergency team activation criteria: A case-control study. *Resuscitation,* 2007, vol. 73 (1), 62-72
- **J.F. FIESELMANN ; M.S. HENDRYX ; C.M. HELMS ; D.S. WAKEFIELD.** Respiratory rate predicts cardiopulmonary arrest for internal medicine inpatients. *Journal of general internal medicine,* 1993, vol. 8 (7), 354-360
- **H. GAO ; A. MCDONNELL ; D.A. HARRISON ; T. MOORE ; S. ADAM ; K. DALY ; L. ESMONDE ; D.R. GOLDHILL ; G.J. PARRY ; A. RASHIDIAN et al.** Systematic review and evaluation of physiological track and trigger warning systems for identifying at-risk patients on the ward. *Intensive care medicine,* 2007, vol. 33 (4), 667-679
- **GOLDBERGER, A. L. et al.** Physiobank, physiotoolkit, and physionet. *Circulation,* 13 June 2000, vol. 101 (23), e215-e220
- **JA HIRSCH ; B. BISHOP.** Respiratory sinus arrhythmia in humans: how breathing pattern modulates heart rate. *American Journal of Physiology-Heart and Circulatory Physiology,* 1981, vol. 241 (4), H620-H629

- **N. IYENGAR ; CK PENG ; R. MORIN ; AL GOLDBERGER ; L.A. LIPSITZ.** Age-related alterations in the fractal scaling of cardiac interbeat interval dynamics. *American Journal of Physiology-Regulatory, Integrative and Comparative Physiology,* 1996, vol. 271 (4), R1078-R1084
- **A. JOHANSSON.** Neural network for photoplethysmographic respiratory rate monitoring. *Medical and Biological Engineering and Computing,* 2003, vol. 41 (3), 242-248
- **J. KAUSE ; G. SMITH ; D. PRYTHERCH ; M. PARR ; A. FLABOURIS ; K. HILLMAN et al.** A Comparison of Antecedents to Cardiac Arrests, Deaths and Emergency Intensive Care Admissions in Australia and New Zealand, and the United Kingdom the ACADEMIA study. *Resuscitation,* 2004, vol. 62 (3), 275-282
- **V.H. LARSEN ; P.H. CHRISTENSEN ; H. OXHØJ ; T. BRASK.** Impedance pneumography for long-term monitoring of respiration during sleep in adult males. *Clinical Physiology,* 1984, vol. 4 (4), 333-342
- **J. LEE ; KH CHON.** Respiratory rate extraction via an autoregressive model using the optimal parameter search criterion. *Annals of biomedical engineering,* 2010, vol. 38 (10), 3218-3225
- **C. ORPHANIDOU ; S. FLEMING ; SA SHAH ; L. TARASSENKO.** Data fusion for estimating respiratory rate from a single-lead ecg. *Biomedical Signal Processing and Control,* 2012
- **J. PAN ; W.J. TOMPKINS.** A real-time QRS detection algorithm. *Biomedical Engineering, IEEE Transactions on,* 1985, vol. 3, 230-236
- **C.E. RASMUSSEN ; C.K.I. WILLIAMS.** Gaussian processes for machine learning. MIT press Cambridge, 2006, vol. 1
- **H. RUE ; S. MARTINO ; N. CHOPIN.** Approximate Bayesian inference for latent Gaussian models by using integrated nested Laplace approximations. *Journal of the royal statistical society: Series b (statistical methodology),* 2009, vol. 71 (2), 319-392
- **N. SHAMIM ; M. ATUL ; C. GARI D et al.** Data fusion for improved respiration rate estimation. *EURASIP Journal on advances in signal processing,* 2010